(19) Europäisches Patentamt
European Patent Office
Office européen des brevets

(11) **EP 4 603 860 A1**

(12) **EUROPEAN PATENT APPLICATION**

(43) Date of publication:
**20.08.2025 Bulletin 2025/34**

(21) Application number: **24157550.5**

(22) Date of filing: **14.02.2024**

(51) International Patent Classification (IPC):
**G01R 33/565** (2006.01) **A61B 5/055** (2006.01)
**A61B 5/00** (2006.01) **G01R 33/561** (2006.01)

(52) Cooperative Patent Classification (CPC):
**G01R 33/56509; A61B 5/055; A61B 5/721;**
G01R 33/5611

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Siemens Healthineers AG**
**91301 Forchheim (DE)**
• **King's College London**
**London WC2R 2LS (GB)**

(72) Inventors:
• **Brackenier, Yannick**
**London, W2 4DQ (GB)**
• **Hajnal, Joseph V.**
**London, NW34PY (GB)**
• **Malik, Shaihan**
**London, N20 9LL (GB)**
• **McElroy, Sarah**
**London, N16 5UD (GB)**

(74) Representative: **Siemens Healthineers
Patent Attorneys
Postfach 22 16 34
80506 München (DE)**

(54) **METHOD FOR RETROSPECTIVELY CALIBRATING AN EXTERNAL MOTION SIGNAL ACQUIRED IN PARALLEL TO AN MRI EXAMINATION**

(57) The invention is directed to a method for retrospectively calibrating an external motion signal (12) in parallel to a magnetic resonance imaging examination (2) of a subject, wherein the magnetic resonance imaging examination (2) comprises several magnetic resonance imaging scans (7), the method comprising the steps of: (a) acquiring a plurality of motion calibration k-space data packets (6) using a magnetic resonance imaging protocol in between the magnetic resonance imaging scans (7); (b) combining the k-space data packets (6) acquired across the magnetic resonance imaging examination (2) and applying an optimization algorithm to the combined k-space data packets (6) in order to estimate motion states of the subject during acquisition of the k-space data packets (6); (c) Estimating a calibration motion model from the motion states estimated in step (b) and the external motion signal (12) acquired simultaneously with the data packets (6), wherein the calibration motion model maps the external motion signal (12) to a corresponding motion state.

FIG 2

**Description**

The present invention relates to a method for retrospectively calibrating an external motion signal acquired in parallel to a magnetic resonance imaging examination, a magnetic resonance imaging apparatus and a computer program.

Independent of the grammatical term usage, individuals with male, female or other gender identities are included within the term.

Patient motion can degrade the diagnostic quality of magnetic resonance (MR) exams.

Some magnetic resonance imaging (MRI) motion correction techniques involve measuring the subject motion by external motion sensors or tracking devices. Such external motion measurements can be used for prospective or retrospective motion-correction of MRI data.

In prospective motion correction, the patient motion is detected in real time and the MRI scanner acquisition geometry is updated during the acquisition to keep the patient at the same position in the imaging coordinate system.

By contrast, retrospective motion correction methods correct for motion artefacts after the data acquisition. This may be done be using data from an external motion measurement during the image reconstruction, or the image data itself may be used to infer both a motion trace of the subject and an estimate of a motion-corrected reconstructed image. Mathematically, this is often done by including a motion operator into the MR forward model. In other words, these so-called data-driven retrospective techniques account for the patient's motion in the final image reconstruction and therefore reduce motion artefacts through improved model agreement. Examples of data-driven retrospective correction techniques using an MR forward model are described in L. Cordero-Grande, E. J. Hughes, J. Hutter, A. N. Price, and J. V Hajnal, "Three-dimensional motion corrected sensitivity encoding reconstruction for multi-shot multi-slice MRI: Application to neonatal brain imaging," Magn. Reson. Med., vol. 79, no. 3, pp. 1365-1376, 2018. For volumetric encoding using array receiver coils and Cartesian sampling, the so-called "aligned SENSE framework" described in J. Cordero-Grande, L., Teixeira, R., Hughes, E., Hutter, J., Price, A., & Hajnal, "Sensitivity Encoding for Aligned Multishot Magnetic Resonance Reconstruction," IEEE Trans. Comput. Imaging, vol. 2, no. 3, pp. 266-280, 2016, achieves motion correction by dividing k-space readouts into temporal segments (shots) and allowing each segment to have a distinct motion state correction that can be estimated.

It has been found that the robustness of retrospective motion estimation can be further improved by using optimized encoding reorderings for rectilinear three-dimensional (3D) multi-shot acquisitions. For example, L. Cordero-Grande, G. Ferrazzi, R. P. A. G. Teixeira, J. O'Muircheartaigh, A. N. Price, and J. V. Hajnal, "Motion-corrected MRI with DISORDER: Distributed and incoherent sample orders for reconstruction deblurring using encoding redundancy," Magn. Reson. Med., vol. 84, pp.713-726,2020, propose a jittered checkerboard reordering scheme for 3D acquisitions (referred to herein as "DISORDER" or "checkered"). By uniformly distributing the samples of each shot across k-space, this reordering was shown to have computational advantages, including improved convergence stability and speed. Another possibility is to additionally acquire k-space data that samples the relevant part of k-space for motion estimation.

Various MRI-compatible external motion measurement systems exist, for example optical or radio-frequency (RF) based methods. Optical methods usually involve one or more cameras, often combined with targets affixed to the subject. The motion of the targets is captured by the camera and thereby, subject motion may be deduced from the optical images. One example of an RF based method is the so-called "Pilot Tone" method. The principle of Pilot Tone is to irradiate the body with an RF signal just outside the receive bandwidth of the MR data being acquired, but within the frequency range of the MR receive system, in particular within the oversampling bandwidth which is acquired during every readout. The Pilot Tone signal interacts with the human body and is received via multiple antennas, for example the local RF coil(s). The received multi-channel Pilot Tone signal can be analysed in order to extract motion information. Other RF-based approaches exist, which often require additional hardware on the receive side.

For the pilot tone and other external motion measurement systems, the received signals (e.g. pixel values from optical cameras, RF signal from pilot tone, etc.) cannot usually be directly used to correct for motion, because they do not record the motion parameters directly, for example as rigid translation and rotation parameters. The measurements must first be calibrated to the actual motion parameters.

One solution would be to execute a training phase in which MRI and the external motion sensor simultaneously acquire data, while the subject and therefore the anatomy of interest assumes different positions. However, this requires additional scan time. Moreover, one has to ask the subject to assume different positions and therefore, this approach does not necessarily capture the full range of poses observed across the examination, and requires the subject to deliberately move, which may not be appropriate for a clinical scenario. Further, the calibration from the training phase must be assumed to remain valid for the data acquired throughout the remainder of the examination. This assumption might break down when the training phase and acquisition are distant in time.

It is therefore an object of the invention to provide a method for calibrating an external motion signal acquired in parallel to an MRI examination, which is robust and does not require patient cooperation.

This object is solved by a method according to claim 1, a magnetic resonance imaging apparatus according to claim 14 and a computer program according to claim 15. Preferred embodiments are given in the dependent claims.

The invention provides a method for retrospectively calibrating an external motion signal acquired by an external motion sensor in parallel to a magnetic resonance imaging examination of a subject, wherein the magnetic resonance imaging examination comprises several magnetic resonance imaging scans, the method comprising the steps of:

(a) acquiring a plurality of motion calibration k-space data packets using a magnetic resonance imaging protocol in between the magnetic resonance imaging scans;
(b) combining the k-space data packets acquired across the magnetic resonance imaging examination and applying an optimization algorithm to the combined k-space data packets in order to estimate motion states of the subject during acquisition of the k-space data packets;
(c) Estimating a calibration motion model from the motion states estimated in step (b) and the external motion signals acquired simultaneously with the data packets, wherein the calibration motion model maps the external motion signal to a corresponding motion state.

The proposed method comprises a novel approach to calibration of external motion sensors in magnetic resonance imaging (MRI). A plurality of motion calibration k-space data packets are acquired in-between the diagnostic magnetic resonance scans. These motion calibration k-space data packets are also referred to as "distributed motion calibration" (DMC) data, because they are acquired in a distributed fashion across the entire examination. By "examination" preferably an imaging session is meant, in which a subject - in particular a patient - is positioned on a patient bed, such that the anatomy of interest is inside the sensitive volume of a magnetic resonance imaging apparatus, e.g. inside the bore of an MRI main magnet. An MRI examination usually comprises one or more adjustment scans, an overview imaging scan often referred to as scout scan, and one or more (diagnostic) magnetic resonance (MR) scans. Usually, about two to ten MR scans are performed, for example to acquire images of the anatomy of interest with different contrasts, and/or before and after the administration of contrast agent. The MR scans, also called diagnostic MR scans, serve to acquire the diagnostic image data which is the purpose of the MR examination. The DMC data according to the present invention is acquired in-between the diagnostic MR scans, in particular in dead-times where for example the next MR scan is being planned, or the operator is talking to the patient to ensure his/her continued well-being inside the MRI apparatus. Preferably, the DMC data consists of fast, motion-sensitized k-space samples that are acquired in a distributed fashion across the entire examination. An external motion signal is acquired at the same time as the DMC data, preferably across the entire examination as well. The DMC data is acquired for the sole purpose of estimating motion parameters, the imaging information contained therein is preferably not used at all. The motion states estimated from the DMC data can then be used to calibrate the external motion sensor. The distributed nature of the DMC acquisitions ensures that all relevant levels of motion occurring during the examination are captured. The DMC data can either be identical acquisitions each time, or they can vary, such they complement each other when retrospectively combined.

In order to improve the estimation of the motion states from the DMC data, all DMC acquisitions across the entire MRI examination are combined into a single estimation algorithm.

Thereby, data-consistency across all DMC acquisitions is enhanced. This improves the robustness of the estimation process.

The invention thus uses a joint estimation from unintrusively sampled data, namely the plurality of motion calibration k-space data packets, also referred to as DMC data, to obtain accurate and comprehensive motion information across the examination, that can be used for calibration of an external motion sensor acquiring an external motion signal. The invention has at least the following three advantages:

1. Instruction-free: Since the DMC data is acquired in a distributed way, and hence distant in time, the full range of patient motion is likely to be captured across all acquisitions. This is in contrast with pre-calibrated training steps as described above. The range of motion detected will also naturally match the range to be corrected - this is helpful for an optimised calibration model.

2. Efficient: DMC acquisitions can be obtained quickly in dead times within the examination, and thus without time penalty and without changing the main examination: The operator selects whatever MR scans they need for the diagnostic questions and the DMC acquisitions sit in the gaps that exist anyway.

3. Robust: Combining all DMC data into a single estimation process (step (b)) will generate robust motion estimation. The use of data interleaved with the full examination means that the calibration is intrinsically valid for the complete data set (no calibration drift). Both of these contrast with prior art approaches.

Because the DMC data are combined across the MRI examination, the external motion signal is calibrated retrospectively. Therefore, the external motion signal is preferably used for retrospective motion correction, for example, using the data-driven retrospective correction techniques using an MR forward model as described above.

The external motion signal can be acquired by any external motion sensor. In particular, the external motion sensors may comprise optical cameras, nuclear magnetic resonance (NMR) probes, electroencephalography-based markers, infrared-cameras, ultrasound-probes or RF-antennas. Optical cameras and RF-antennas are preferred. An optical camera may for example acquire an external motion signal in the form of a video footage of the patient from one or more video cameras mounted inside or at the edge of the sensitive area of the MRI apparatus, for example at the entrance to the bore or inside the bore of a main magnet. The RF-antenna may be a separate antenna from the RF coil used to acquire the MR signals. Preferably, it is an RF coil used to acquire MR signals. Preferably, the external motion signal is a pilot tone (PT) signal. For the PT method, an RF signal just outside the bandwidth of the MRI examination, but inside the receive bandwidth of the RF coils, is radiated into the sensitive area of the MR scanner, for example, by an additional RF-antenna. The RF signal interacts with the subject's body and varies therefore with the subject's motion. It is picked up by the MR receiver coil or coils, preferably by an array coil having several channels. Because the PT and MR signals are acquired using the same receiver system, without interfering with each other since they occupy different frequency bands, the external motion signals and the MR signals, in particular the motion calibration k-space data packets, are automatically synchronized. This, in addition to its quick and easy workflow setup, makes PT a preferred candidate for the external notion signal, although the invention is not limited thereto.

The magnetic resonance scans may be acquired using any known imaging protocol. They may, for example, comprise two-dimensional (2D) and/or three-dimensional (3D) scans. They may be acquired using any imaging protocol, for example spin-echo based or gradient-echo based imaging protocols. There is no limitation on the type of imaging protocol that can be used for the several magnetic resonance scans acquired during the MRI examination, because these scans are not modified in any way by the inventive method. The inventive method only aims at acquiring additional data, namely the motion calibration k-space data packets in-between the MR scans. To this end, it may be possible that the control unit of the MR apparatus (also referred to as MR scanner) issues a trigger signal every time an MR scan is finished. The trigger signal can be used to start the acquisition of the motion calibration k-space data packets. This DMC acquisition may either have a fixed duration, or the acquisition may continue for as long as the next MR scan is not started. In this embodiment, when the operator, e.g. an MR technician, has finished planning the next MR scan and starts the acquisition thereof, the acquisition of the DMC data is automatically interrupted, until the end of the next MR scan. Thereby, k-space data packets for motion calibration can be acquired in-between the several MR scans throughout the MRI examination.

In step (b), which is performed at the end of the MRI examination, the DMC data acquired across the MRI examination are combined. An optimization algorithm is applied in order to estimate the motion states of the subject during acquisition of the DMC data. A "motion state" may in particular comprise positional information of the anatomy of interest, e.g. the volume from which the MR scans and the DMC data is acquired. In a preferred embodiment, the motion states are expressed by rigid body motion parameters, preferably by three rotational and three translational parameters. However, the invention is not limited thereto, and non-rigid motion may also be estimated using the method of the invention. Several motion states detected over time are referred to herein as "motion trace". The motion parameters can be estimated from the DMC data using known methods such as a perturbation model, or a scout-based model. A perturbation-based model is described for example in Thomas Ulrich, Malte Riedel, Klaas P. Pruess-mann "Servo navigators: Linear regression and feedback control for rigid-body motion correction", Magn. Reson. Med., online version first published on 17 January 2024, https://doi.org/10.1002/mrm.29967. In a scout-based method, the k-space data packets are essentially compared against a low-resolution scout reference image, which may be the scout image which is acquired in order to plan the MR scans at the beginning of the MR examination. It may also be an additional scout image acquired at the beginning of the imaging scan. This method is described in D. Polak et al. "Scout accelerated motion estimation and reduction (SAMER)", Magn. Reson. Med., vol. 87, pp. 163-178, 2022, and which is incorporated herein by reference.

Given a total number N of DMC acquisitions, $\sum_{n=1}^{N} M_n \mathbb{M}$ motion states will be available for the calibration step, with $M_n$ the number of motion states within each DMC **n**. When the external motion signal is received using D channels, a calibration motion model may be estimated in step (c), which maps from the external motion signal to the motion states of the subject. No restrictions apply to the type of model that can be used. In a preferred embodiment, a linear model is used, which comprises a calibration matrix C, which maps between both domains. An example of the underlying mathematics is given herein below.

According to a preferred embodiment, the calibration motion model is thus a linear model and comprises a calibration matrix which maps an external motion signal on a motion state of the subject. This is a useful simplification which makes the estimation step stable and computationally robust.

Further, according to an embodiment, the external motion signal is a pilot tone signal, in particular, a multi-channel pilot tone signal acquired with a magnetic resonance receiver coil array. The PT has the advantage that it requires no or little additional hardware.

According to an embodiment, the acquisition of the k-space data packets is distributed across a significant portion of the subject's magnetic resonance imaging examination, in particular over more than half, preferably more than ¾

of the examination.

Most preferred, the acquisition of the k-space data packets (DMC data) is distributed across the complete MRI examination. This has the advantage that all kinds of relevant motion of the subject, which may occur throughout the examination, will be captured in the DMC data. It may be achieved by triggering a DMC acquisition after each MR scan, and possibly also after the initial adjustment and/or scout scans.

According to an embodiment, the k-space data packets are acquired in the dead times between the magnetic resonance imaging scans, and wherein preferably the duration of acquisition of each k-space data packet is adapted to the duration of the dead time between the respective magnetic resonance imaging scans. By "dead time" we mean the times that are typically required in an MRI examination in-between the MR scans, for checking the quality of the MR scan that has just been completed, and/or for planning the next MR scan, and/or for injecting contrast agent and/or for communicating with the subjects to ensure his/her well-being. The dead time is time that is usually unused, and is now used to acquire the motion calibration k-space data packets. The length of acquisition of each k-space data packet may vary from acquisition to acquisition, according to the length of the dead time available. In other embodiments, the k-space data packets all have the same, pre-set duration. The duration may for example be between 0.3 and 30 seconds, preferably 5-20 seconds.

According to an embodiment, the method comprises a further step of using the calibration motion model and the acquired external motion signal for retrospective motion correction of the data acquired in the magnetic resonance imaging scans.

Thereby, the MRI scans may be retrospectively motion corrected. To achieve this, known retrospective data deconstruc-tion methods may be used, for example, techniques using an MR forward model described by L. Cordero-Grande mentioned above. Thus, the method of this embodiment is essentially a method for acquiring motion-corrected magnetic resonance imaging data.

The motion-corrected MRI data may be obtained by two possible embodiments.

According to a first embodiment, the method comprises a step of obtaining a motion trace of the subject, by applying the calibration motion model to the external motion signal acquired throughout the magnetic resonance imaging examination, and using the motion trace in retrospective motion correction of the data acquired in the magnetic resonance imaging scans. Thus, the motion trace of the subject is estimated first, namely by applying the calibration motion model to the external motion signal acquired throughout the MRI examination and in particular during the MR scans. This motion trace comprising motion states during the acquisition of the MR scans, is then used to retrospectively motion correct the data acquired in the MR scans. This has the advantage that it is easy to apply, however, it assumes that the calibration motion model remains the same throughout the MRI examination. Since this may not always hold true, another embodiment is also proposed:

According to another embodiment, the method comprises a further step of reconstructing images from the k-space data acquired in the magnetic resonance imaging scans by minimizing the data consistency error between the k-space data acquired in the magnetic resonance imaging scans and an image reconstruction forward model described by an encoding matrix, wherein the encoding matrix includes motion states of the subject, optionally the sensitivity profiles (S) of a receiver coil array, Fourier encoding (F), and a sampling mask (A), and wherein the motion states in the encoding matrix are obtained by using a motion model applied to the external motion signal, and wherein the calibration motion model from claim 1 is used as an initial estimate for the motion model.

In this embodiment, essentially both the motion trace and the motion-corrected images are estimated in a single step. The calibration motion model is not even absolutely required, in particular, in the event that the MR scans are acquired using a distributed reordering scheme, wherein each shot covers k-space samples in the center of k-space, an initial estimate for the motion model may not be required. Usually, however, the MR scans will use normal, sequential k-space sampling schemes because these yield better results in other respects. In this case, it is advantageous to use the calibration motion model estimated from the motion states extracted from the combined k-space data packets as an initial estimate for the motion model. An example for a mathematical description of the image reconstruction forward model is given herein below. This embodiment has the advantage that the data-consistency across all acquisitions is maximized. Thus, retrospective joint motion and image estimation leverages the data-consistency between DMC data. Therefore, it may take into account such cases where the calibration motion model changes during the MR examination.

According to an embodiment, the motion calibration k-space data packets are acquired using a low-resolution three-dimensional imaging protocol, and wherein the acquisition of one three-dimensional image is distributed over several data packets, or is completed in one data packet. For example, the 3D imaging protocol may be a gradient echo imaging protocol. Low resolution may mean more than $2{\times}2{\times}2mm^3$ resolution, preferably up to $6{\times}6{\times}6mm^3$ resolution. In the example, experiments were performed at $4{\times}4{\times}4mm^3$ resolution. The total acquisition time to acquire a full k-space in this imaging protocol may be between 1.0 and 100 secs, preferably between 2.0 and 60 secs, more preferred between 5 and 40secs. In the example acquisitions, the sequence parameters were TE / TR = 1.93 / 3.8ms, flip angle 8°, scan duration 22s. Such a 3D scan may be acquired as one k-space data packet. This has been done in the example, but it is not necessary. Alternatively, it may be preferred to distribute the acquisition of a 3D image over several k-space data packets. This gives more flexibility to use even shorter dead times between MR scans. In an extreme embodiment, a single k-space would only be fully sampled by the end of the examination.

According to an embodiment, each k-space data packet is divided into one or more temporal segments, wherein the calibration motion model comprises a motion state for each temporal segment. A temporal segment preferably corresponds to a shot, wherein several k-space samples are acquired in each shot. For example, each calibration k-space data packets comprises a plurality of shots, wherein several k-space lines are acquired in one shot. An "shot", also referred to as "echo train", comprises a plurality of magnetic resonance (MR) echoes, e.g. spin echoes and/or gradient echoes. During each echo, a line in k-space is sampled (termed "k-space sample". In most sequences, a shot comprises a preparation pulse, and then all echoes have their own excitation/refocus-sing pulses. Thus, a shot may mean a series of gradient echoes or spin echoes, each echo corresponding to one line in k-space. There may be 8 to 512 echoes in one shot, wherein the acquisition duration of a shot may be between 0.1 and 4s, preferably between 0.2 and 1s. It has been found useful to estimate one motion state per shot, because this results in a time resolution which is useful to capture most patient movements. Each k-space data packet may comprise for example between 1 and 30 shots. The duration of each temporal segment is for example between 0.1sec and 3.0 sec, preferably between 0.2 and 1.2sec, more preferred between 0.3 and 0.6 sec. Thereby, high temporal resolution motion estimates may be achieved. Further, this embodiment has the advantage that the temporal resolution for the motion states may be determined by the user by determining the length of the temporal segments which are used in the optimization algorithm to represent one motion state.

According to an embodiment, motion calibration k-space data packets are acquired using a distributed sampling order, in which samples acquired during one temporal segment are distributed across k-space, wherein in particular successively acquired k-space samples are not adjacent to each other in k-space. In a 3D-imaging protocol, a thick slab of tissue is excited together. In such 3D-imaging, spatial encoding may be performed using phase-encoding gradients along two spatial dimensions, and frequency encoding along the third spatial dimension, referred to as readout direction. Accordingly, with each MR echo in a shot, a k-space sample oriented along the readout direction is sampled. By modifying the phase-encoding gradient before each readout, it is possible to design different sampling orders (also referred to as "reordering", "sampling order", or "reordering scheme"), which may refer to the order in which the k-space samples are acquired. For example, in a linear reordering, the phase-encoding gradient is increased step by step for each k-space sample, resulting in a sampling of k-space line-by-line, from one end of k-space to the other. However, a distributed sampling scheme, for example as disclosed by Cordero-Grande in MRM 2020 ("DISORER", full reference see above) is preferred, because thereby the k-space data packets are effectively motion-sensitized. This is because patient motion is reflected in different areas of k-space to different degrees. It has been shown that it is preferable that a central region of k-space should be sampled in each time segment, so as to best capture patient motion in the DMC data. Therefore, distributed sampling schemes are preferred.

According to an embodiment, 3 to 20, preferably 5 to 10 motion calibration k-space data packets are acquired throughout the magnetic resonance imaging examination, and/or wherein each motion calibration k-space data packet is acquired during 1 to 200 sec, preferably during 5 to 100 sec, more preferred 10 to 60 sec. It has been shown that this amount of DMC data is sufficient to achieve a satisfactory calibration of the external motion sensors.

According to an embodiment, the optimization algorithm used to estimate motion states of the subject from the acquired k-space data packets includes a step of minimizing the data consistency error between the k-space data of the data packets and an image reconstruction forward model described by an encoding matrix, wherein the encoding matrix includes motion states of the subject, optionally the sensitivity profiles of a receiver coil array, Fourier encoding and a sampling mask. For example, the PT-aligned SENSE construction model may be used, as described herein below. However, the invention is not limited thereto, other optimization algorithms may be used. For example, other optimization algorithms could use the "perturbation model" (see above) or methods using the image-equivalent of the acquired k-space samples within a shot and performing image registration. An example of this method is described by Emil Ljungberg et al. in "Motion corrected silent ZTE neuroimaging", Magn. Reson. Med, Vol. 88, pp. 195-210 (2022)

In a further aspect of the invention, a magnetic resonance imaging apparatus is provided which comprises a radio frequency controller configured to drive an RF-coil, preferably comprising a multi-channel coil-array, a gradient controller configured to control gradient coils, and a control unit configured to control the radio frequency controller and the gradient controller to execute the imaging protocol according to the invention. The MRI-apparatus may be a commercially available MRI-apparatus which has been programmed to perform the method of the invention.

All advantages and features of the inventive method may also apply to the MRI apparatus and vice versa. Generally, the MRI apparatus may comprise a MR scanner. Apart from the external motion sensor, the MRI apparatus does not require additional hardware in order to execute the inventive method.

According to a further aspect of the invention, a computer program is provided which includes program code, which causes a magnetic resonance imaging apparatus - such as the apparatus described herein - to execute the inventive method, in particular the inventive method for acquiring an MR image dataset. However, the program code may also encode the described method for generating a motion-corrected magnetic resonance image dataset, and the program code may run on a computer as described herein.

According to a further aspect, the invention is directed to a non-transitory computer-readable medium containing a computer program as described herein. The computer-readable medium may be any digital storage medium, such as a hard disc, a cloud, an optical medium such as a CD or DVD, a memory card such as a compact flash, memory stick, a USB-stick, multimedia stick, secure digital memory card (SD) etc.

All advantages and features given for the MRI apparatus and the inventive method also apply to the computer program, computer program product, digital storage medium and vice versa. The computer program may be executed by any MRI imaging apparatus which may receive the signal of an external motion sensor, for example a pilot tone signal.

In the following, a mathematical description of an example embodiment of the optimization algorithm and estimation step of the present invention is given. However, the invention is not limited to this model.

Both the Pilot Tone signal and the MRI data are acquired using an array receiver coil having a number of D channels. In this example, it is a head coil and the anatomy of interest is the head. The motion to be corrected is assumed to be rigid motion. Evidently, similar mathematics may be used to describe other external motion signals.

To describe the variation of the PT signal with head position, one assumes a linear relationship between the 6 rigid motion parameters (3 rotational and 3 translational parameters) ($z_t$) and the PT signal at time t ($p_t$), which has D channels, D being the number of channels of the coil receiver array:

$$z_t = C\, p_t \qquad [1]$$

where $p_t$ is a complex vector containing the D-channel PT signal at time $t$. The PT signal is preferably concatenated with a 1 to allow for a motion offset. $C$ is a 6x(D+1) matrix with calibration coefficients for each of the 6 rigid motion parameters.

Thus, the calibration matrix $C$ is an example of a calibration motion model which maps the external motion signal at time t, $p_c$, to a corresponding motion state $z_t$.

An example using an MR forward model is the alignedSENSE reconstruction problem (J. Cordero-Grande, L., Teixeira, R., Hughes, E., Hutter, J., Price, A., & Hajnal, "Sensitivity Encoding for Aligned Multishot Magnetic Resonance Reconstruction," IEEE Trans. Comput. Imaging, vol. 2, no. 3, pp. 266-280, 2016) with rigid motion correction and parallel imaging, which can be formulated as an inverse problem in matrix form:

$$(\hat{x}, \widehat{z_n}) = argmin_{x,z_n} \sum_n \left\| A_n F\, S\, T_{z_n}\, x - y_n \right\|_2^2 \qquad [2]$$

Where $x$ is the volumetric image to be reconstructed, $S$ is the sensitivity profile of the D-element coil receiver array and $F$ the discrete Fourier transform. For each temporal segment or shot $n$, $T_{z_n}$ is the rigid motion operator with motion parameters $z_n$, $A_n$ the sampling mask and $y_n$ the acquired k-space data of the imaging scan for all coil elements.

By inserting equation 1 into equation 2, the alignedSENSE reconstruction problem can be expanded to include the Pilot Tone signal $p$ as an input, and to estimate the calibration matrix $C$ together with the reconstructed image $x$. $p_n$ as the averaged PT signal at shot $n$, and inserting equation 1 into equation 2 results in the following inverse problem, referred to as PT-alignedSENSE:

$$(\hat{x}, \hat{C}) = argmin_{x,C} \ \sum_n \left\| A_n F S T_{Cp_n} x - y_n \right\|_2^2 \qquad [3]$$

Equation 3 can be solved by iteratively updating $x$ and $C$, for example using Conjugate Gradient (CG) and Levenberg-Marquardt (LM) algorithms, respectively.

Processing of the DMC acquisitions

Preferably, a pre-calibrated $C$, $C_{calib}$, is used to solve equation [3]. $C_{calib}$ can be estimated using the present invention, in particular from the k-space data packets, also referred to as DMC acquisitions, and a set of motion states of the subject during the acquisition of the k-space data packets. This is in particular preferred for standard, sequential sampling schemes of the MR scans, because for those standard, sequential sampling schemes, equation [3] would not yield satisfactory results. Rather, robust performance would require the use of distributed sampling schemes (like DISORDER, VD-CASPER, Linear+, etc) in the MR scans, in which each shot collects samples spanning across k-space. That is possible, but required modification of existing MR scan protocols. The invention is however designed to work with any MR scan protocols.

Therefore, the invention distributes individual motion calibration (MC) k-space data packets acquisitions $y_{MC}$ throughout the examination. After combining all MC acquisitions into $y_{DMC}$, motion parameters $z_{DCM_m}$ corresponding to

each shot $y_{DCM_m}$ are obtained using the alignedSENSE reconstruction (Equation 2):

$$(\widehat{x_{DMC}}, \widehat{z_{DCM_m}}) = argmin_{x,z_{DCM_m}} \sum_m \left\| A_m F S T_{z_{DCM_m}} x_{DMC} - y_{DCM,m} \right\|_2^2 \quad [4]$$

In preferred embodiments, multiple shots *m* may be contained within a single $y_{MC}$. To enable robust motion estimation, a self-navigated (=distributed) reordering scheme is preferably used for all motion calibration (MC) k-space data packets. Since the distributed motion calibration k-space data packets acquisitions are only used for motion estimation, acquisitions are low-resolution and the estimated image $x_{DMC}$ is not used. After the motion parameters $z_{DCM_m}$ are obtained and the simultaneously acquired PT signals $p_{DCM_m}$ are extracted from the acquired MR data, $\widehat{C_{calib}}$ can be estimated using a least-squares fit:

$$(\widehat{C_{calib}}) = argmin_C \sum_m \left\| z_{DMC,m} - C\, p_{DMC,m} \right\|_2^2 \quad [5]$$

This $\widehat{C_{calib}}$ can be used as an initial estimate in equation 3, in order to iteratively estimate the motion corrected images for the MR scans.

Alternatively, $\widehat{C_{calib}}$ as estimated from equation 5 can be taken as the final calibration motion model which is applied to the external motion signal *p* to obtain a motion trace, which in turn is used for retrospective motion correctio of the MR data acquired in the MR scans.

The invention will now be described by means of embodiments with reference to the attached drawings. In the drawings:

Fig. 1 shows a timeline of an embodiment of the present invention;

Fig. 2 shows a flow diagram of an embodiment of the inventive method;

Fig. 3 shows an embodiment of an MRI apparatus according to the invention.

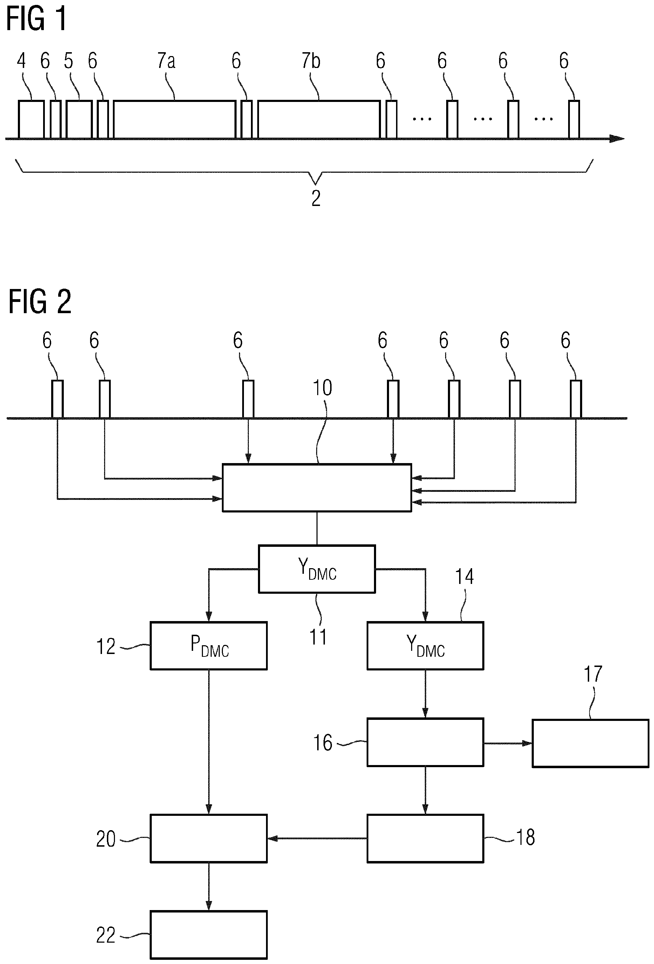

Fig. 1 illustrates the timeline of an MRI examination according to an embodiment of the inventive method. The entire examination 2 might take between 15 and 70 minutes, preferably between 20 and 50 minutes. It is constituted by the timespan that the subject or patient stays inside the sensitive area or bore of the MRI apparatus. At the beginning, there will usually be a scout scan 4, which is a very fast, low-resolution 2D or 3D imaging scan, which is taken to provide the operator with an overview of the anatomy of interest, and an image on which the operator plans the further diagnostic scans 6. The scout may be followed by a reference scan 5. However, in between the scout and resonance scan, a DMC acquisition 6 may be placed. As mentioned above, the DMC acquisition 6 may for example be triggered at the end of the scout scan 4, and may be carried up to a maximum pre-determined duration, unless another scan is initiated by the operator during the DMC acquisition in which case it may be interrupted. In another embodiment, the DMC acquisition 6 is a fixed type of acquisition having a fixed length, which cannot be interrupted by further scans. For example, the DMC acquisition 6 may be a low-resolution 3D imaging scan having a duration of between 15 and 40 seconds and comprising 20-100 shots. After the reference scan 5, several diagnostic imaging scans 7a and 7b are carried out. In between, again DMC acquisition 6 are placed, wherein k-space data packets are acquired in order to estimate motion states of the subject throughout the MRI examination 2.

Fig. 2 illustrates the processing of the DMC acquisition 6 according to an embodiment of the invention. In step 10, the DMC data packets are combined, preferably concatenated, into one data set termed $y_{DMC}$, which comprises all k-space data acquired during the DMC acquisition 6. In this embodiment, the external motion signal is a pilot tone signal, which is acquired together with the MR signal. Therefore, the method includes a step 11 of extracting the pilot tone signal $p_{DMC}$ 12 from the combined DMC data. The further processing is done with the $y_{DMC}$ data 14. In the next step 16, which may for example use an optimisation algorithm to optimise equation 3, the motion parameters $z_{DMC}$ 18 are estimated from the combined DMC data 14. A motion corrected image 17 is also the result of this optimisation, but is not used in the further processing. The motion trace 18, which may for example comprise rigid body motion parameters for each shot of the DMC acquisitions, is used instead in step 20, in order to find out the calibration motion model 22. Step 20 may for example be carried out using the mathematical model of equation 5. However, other mathematical descriptions/models are also possible.

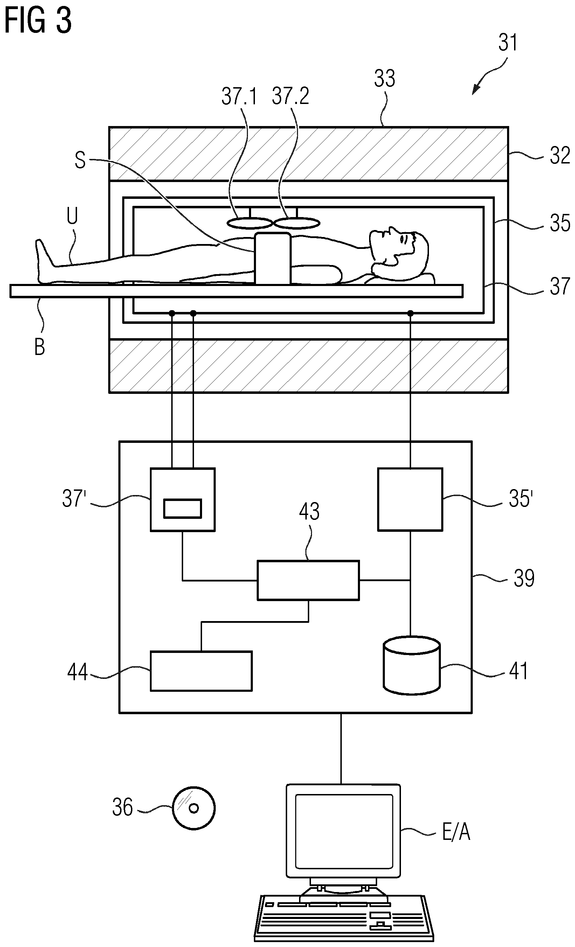

Fig. 3 schematically shows an inventive magnetic resonance (MR) apparatus 31. The MR apparatus 31 has an MR data acquisition scanner 32 with a magnet 33 that generates the constant magnetic field, a gradient coil arrangement 35 that generates the gradient fields, one or several radio-frequency (RF) antennas 37 for radiating and receiving RF signals, and a control computer 39 configured to perform the inventive method. The radio-frequency antennas 37 may include a multi-channel coil array comprising at least two coils, for example the schematically shown coils 37.1 and 37.2, which may be configured to transmit and/or receive RF signals (MR signals) .

In order to acquire MR data from an examination subject U, for example a patient or a phantom, the subject U is introduced on a bed B into the measurement volume of the scanner 32. MR data can be acquired using a method according to an embodiment of the invention from 3D slab S. The control computer 39 controls the MR apparatus 1, and the gradient coil arrangement 35 with a gradient controller 35' and the RF antenna 37 with a RF transmit/receive controller 37'. The RF antenna 37 has multiple channels corresponding to the multiple coils 37.1, 37.2 of the coil arrays, in which signals can be transmitted or received. The control computer 39 also has an imaging protocol processor 44 that determines the imaging protocol, including the sampling order and the acquisition of the k-space data packets. A control unit 43 of the control computer 39 is configured to execute all the controls and computation operations required for acquisitions. Intermediate results and final results required for this purpose or determined in the process can be stored in a memory 41 of the control computer 39. A user can enter control commands and/or view displayed results, for example image data, an input/output interface E/A. A non-transitory data storage medium 36 can be loaded into the control computer 39, and may be encoded with programming instructions (program code) that cause the control computer 39, and the various functional units thereof described above, to implement any or all embodiments of the method according to the invention.

A method according to an embodiment of the invention was implemented on a 7T Siemens Terra system, using a 1TX coil (NOVA medical) using a 32-element adult head coil array. MRI examinations were conducted on twelve healthy volunteers, wherein the MRI scans were for example MPRAGE acquisition, some with a DISORDER sampling (Acquisition time per shot=0.76sec), some using a standard linear sampling scheme. The k-space data packets comprised six individual fully sampled DMC acquisitions placed throughout the examination. Each DMC acquisition was a 3D acquisition using am RF + gradient-spoiled gradient echo sequence having a 3x3x3 mm$^3$ resolution, FOV=240x212x240 mm$^3$, TE/TR = 1,93/3.8 ms, flip angle 8°, scan duration 22 s. After each MRI examination, motion parameters were estimated using equation 4 and subsequently used to construct $C_{calib}$ in equation 5. $C_{calib}$ was validated using the DISORDER MPRAGE acquisition. The latter, being a self-navigated sequence, may be considered to achieve ground through motion parameters after motion correction. The external motion signal was the pilot tone signal. Motion parameters are predicted using the PT signals extracted from the DISORDER MPRAGE. The duration of a shot in the example was 0.33sec, achieving high-temporal resolution motion estimates. Using such setup, it was shown to produce effective calibration of motion parameters to the externally acquired motion signal. The calibration motion model obtained using the proposed invention achieved successful motion correction in standard MPRAGE acquisitions in volunteers that were not instructed to move during the calibration scans.

The proposed solution uses data acquired in unused time throughout the examination and samples the range of motions the subject actually does. This means there is no time penalty and the calibration is based on motions that are likely to match those that disrupted the data to be corrected. Also, the calibration model is intrinsically valid for the duration of the examination. Since there is no change in the main examination, the results obtained are the standard results (uncorrected) or better results if the calibration process and motion corrected reconstruction is effective - this gives a non-inferiority guarantee.

**Claims**

1. A Method for retrospectively calibrating an external motion signal (12) in parallel to a magnetic resonance imaging examination (2) of a subject, wherein the magnetic resonance imaging examination (2) comprises several magnetic resonance imaging scans (7), the method comprising the steps of:

    (a) acquiring a plurality of motion calibration k-space data packets (6) using a magnetic resonance imaging protocol in between the magnetic resonance imaging scans (7);
    (b) combining the k-space data packets (6) acquired across the magnetic resonance imaging examination (2) and applying an optimization algorithm to the combined k-space data packets (6) in order to estimate motion states of the subject during acquisition of the k-space data packets (6);
    (c) Estimating a calibration motion model from the motion states estimated in step (b) and the external motion signal (12) acquired simultaneously with the data packets (6), wherein the calibration motion model maps the external motion signal (12) to a corresponding motion state.

2. The method of claim 1, wherein the acquisition of the k-space data packets (6) is distributed across a significant portion of the subject's magnetic resonance imaging examination (2), in particular over more than half, preferably more than ¾ of the examination.

3. The method of claim 1 or 2, wherein the k-space data packets (6) are acquired in the dead times between the magnetic resonance imaging scans (7), and wherein preferably the duration of acquisition of each k-space data packet is adapted to the duration of the dead time between the respective magnetic resonance imaging scans (7) .

4. The method of one of the preceding claims, comprising a further step of using the calibration motion model and the acquired external motion signal (12) for retrospective motion correction of the data acquired in the magnetic resonance imaging scans (7).

5. The method of one of the preceding claims, comprising the further steps of

- obtaining a motion trace of the subject, by applying the calibration motion model to the external motion signal (12) acquired throughout the magnetic resonance imaging examination (2), and
- using the motion trace in retrospective motion correction of the data acquired in the magnetic resonance imaging scans (7).

6. The method of one of the preceding claims 1 to 4, comprising a further step of reconstructing images from the k-space data acquired in the magnetic resonance imaging scans (7) by minimizing the data consistency error between the k-space data acquired in the magnetic resonance imaging scans (7) and an image reconstruction forward model described by an encoding matrix, wherein the encoding matrix includes motion states of the subject, optionally the sensitivity profiles (S) of a receiver coil array, Fourier encoding (F), and a sampling mask (A), and wherein the motion states in the encoding matrix are obtained by using a motion model applied to the external motion signal (12), and wherein the calibration motion model from claim 1 is used as an initial estimate for the motion model.

7. The method of one of the preceding claims, wherein the external motion signal (12) is a pilot tone signal, in particular a multi-channel pilot tone signal acquired with a magnetic resonance receiver coil array.

8. The method of one of the preceding claims, wherein the motion calibration k-space data packets (6) are acquired using a low-resolution three-dimensional imaging protocol, and wherein the acquisition of one three-dimensional image is distributed over several data packets (6), or is completed in one data packet.

9. The method of one of the preceding claims, wherein each k-space data packet is divided into one or more temporal segments, wherein the calibration motion model comprises a motion state for each temporal segment, and wherein a temporal segment preferably corresponds to a shot, wherein several k-space samples are acquired in each shot.

10. The method of one of the preceding claims, wherein the motion calibration k-space data packets (6) are acquired using a distributed sampling order, in which samples acquired during one temporal segment are distributed across k-space, wherein in particular successively acquired k-space samples are not adjacent to each other in k-space.

11. The method of one of the preceding claims, wherein 3 to 20, preferably 5 to 10 motion calibration k-space data packets (6) are acquired throughout the magnetic resonance imaging examination, and/or wherein each motion calibration k-space data packet is acquired during 1 to 200 sec, preferably during 5 to 100 sec, more preferred 10 to 60 sec.

12. The method of one of the preceding claims, wherein the optimization algorithm used to estimate motion states of the subject from the acquired k-space data packets (6) includes a step of minimizing the data consistency error between the k-space data of the data packets (6) and an image reconstruction forward model described by an encoding matrix, wherein the encoding matrix includes motion states of the subject, optionally the sensitivity profiles (S) of a receiver coil array, Fourier encoding (F), and a sampling mask (A).

13. The method of one of the preceding claims, wherein the calibration motion model is a linear model and comprises a calibration matrix which maps an external motion signal (12) on a motion state of the subject.

14. A magnetic resonance imaging apparatus (1) configured to execute the method of one of the preceding claims, comprising:

a radio frequency controller (7') configured to drive an RF-coil (7) comprising a multi-channel coil array (7.1, 7.2),
a gradient controller (5') configured to control gradient coils (5),
a control unit (13) configured to control the radio frequency controller (7') and the gradient controller (5') to execute the method of one of the preceding claims.

15. A computer program including program code, which causes a magnetic resonance imaging apparatus (1) to execute the method according to any one of claims 1 to 13.

# FIG 1

# FIG 2

# FIG 3

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TOM WILKINSON ET AL: "Motion Estimation for Brain Imaging at Ultra-High Field Using Pilot-Tone: Comparison with DISORDER Motion Compensation", PROCEEDINGS OF THE 2021 ISMRM & SMRT ANNUAL MEETING & EXHIBITION, 15-20 MAY 2021, ISMRM, 2030 ADDISON STREET, 7TH FLOOR, BERKELEY, CA 94704 USA, no. 122, 30 April 2021 (2021-04-30), XP040722142, * the whole document * | 1-15 | INV. G01R33/565 A61B5/055 A61B5/00 ADD. G01R33/561 |
| A | YANNICK BRACKENIER ET AL: "Pilot Tone meets DISORDER: Improved data-driven motion-corrected brain MRI by leveraging Pilot Tone signal variations", PROCEEDINGS OF THE JOINT ANNUAL MEETING ISMRM-ESMRMB 2022 & ISMRT ANNUAL MEETING, LONDON, UK, 07-12 MAY 2022, ISMRM, 2030 ADDISON STREET, 7TH FLOOR, BERKELEY, CA 94704 USA, no. 859, 22 April 2022 (2022-04-22), XP040727407, * the whole document * | 1-15 | |
| A | TOM WILKINSON ET AL: "Pilot-Tone Motion Estimation for Brain Imaging at Ultra-High Field Using FatNav Calibration", PROCEEDINGS OF THE JOINT ANNUAL MEETING ISMRM-ESMRMB 2022 & ISMRT ANNUAL MEETING, LONDON, UK, 07-12 MAY 2022, ISMRM, 2030 ADDISON STREET, 7TH FLOOR, BERKELEY, CA 94704 USA, no. 1955, 22 April 2022 (2022-04-22), XP040728503, * the whole document * | 1-15 | TECHNICAL FIELDS SEARCHED (IPC) G01R A61B |

The present search report has been drawn up for all claims

| Place of search | Date of completion of the search | Examiner |
|---|---|---|
| Munich | 1 July 2024 | Lebar, Andrija |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **L. CORDERO-GRANDE** ; **E. J. HUGHES** ; **J. HUTTER** ; **A. N. PRICE** ; **J. V HAJNAL**. Three-dimensional motion corrected sensitivity encoding reconstruction for multi-shot multi-slice MRI: Application to neonatal brain imaging. *Magn. Reson. Med.*, 2018, vol. 79 (3), 1365-1376
- **J. CORDERO-GRANDE, L.** ; **TEIXEIRA, R** ; **HUGHES, E** ; **HUTTER, J.** ; **PRICE, A.** ; **HAJNAL**. Sensitivity Encoding for Aligned Multishot Magnetic Resonance Reconstruction. *IEEE Trans. Comput. Imaging*, 2016, vol. 2 (3), 266-280
- **L. CORDERO-GRANDE** ; **G. FERRAZZI** ; **R. P. A. G. TEIXEIRA** ; **J. O'MUIRCHEARTAIGH** ; **A. N. PRICE** ; **J. V. HAJNAL**. Motion-corrected MRI with DISORDER: Distributed and incoherent sample orders for reconstruction deblurring using encoding redundancy. *Magn. Reson. Med.*, 2020, vol. 84, 713-726
- **THOMAS ULRICH** ; **MALTE RIEDEL** ; **KLAAS P. PRUESS-MANN**. Servo navigators: Linear regression and feedback control for rigid-body motion correction. *Magn. Reson. Med.*, 17 January 2024, https://doi.org/10.1002/mrm.29967
- **D. POLAK et al.** Scout accelerated motion estimation and reduction (SAMER). *Magn. Reson. Med.*, 2022, vol. 87, 163-178
- **EMIL LJUNGBERG et al.** Motion corrected silent ZTE neuroimaging. *Magn. Reson. Med*, 2022, vol. 88, 195-210
- **J. CORDERO-GRANDE, L** ; **TEIXEIRA, R** ; **HUGHES, E** ; **HUTTER, J.** ; **PRICE, A.** ; **HAJNAL**. Sensitivity Encoding for Aligned Multishot Magnetic Resonance Reconstruction. *IEEE Trans. Comput. Imaging*, 2016, vol. 2 (3), 266-280